# EUROPEAN PATENT APPLICATION

(11) **EP 4 458 941 A1**
(43) Date of publication of application: **06.11.2024**
(21) Application number: 21969270.4
(22) Date of filing: 27.12.2021
(51) Int. Cl.: C12M 1/00, C12M 1/34

(54) **MEMBRANE FORMATION APPARATUS AND MEMBRANE FORMATION METHOD**

(71) Applicant: BGI Shenzhen, Shenzhen, Guangdong 518083 (CN)
(72) Inventor: ZHANG, Yuning, SHENZHEN, Guangdong 518083 (CN); YUN, Quanxin, SHENZHEN, Guangdong 518083 (CN); LI, Yuxiang, SHENZHEN, Guangdong 518083 (CN); DONG, Yuliang, SHENZHEN, Guangdong 518083 (CN); ZHANG, Wenwei, SHENZHEN, Guangdong 518083 (CN); XU, Xun, SHENZHEN, Guangdong 518083 (CN)
(74) Representative: Inspicos P/S
(86) International application number: PCT/CN2021/141635
(87) International publication number: WO 2023/122883

(57) **Abstract**

A membrane forming device and a membrane forming method are provided. The membrane forming device includes: a base (10); a main body (20) arranged to the base (10) and the main body (20) having a recess; and an opening member (30) arranged inside a recess port on an end of the recess away from the base (10), and a hollow portion (32) of the opening member (30) being configured to form a amphipathic molecular membrane (80), wherein a cross-sectional area of the hollow portion (32) of the opening member (30) is smaller than that of the accommodating chamber (21).

## Description

### TECHNICAL FIELD

This disclosure relates to the technical field of biopharmaceuticals, and in particular to a membrane forming device and a membrane forming method.

### BACKGROUND

Nanopore-based single-molecule gene sequencing is considered a disruptive technology in the field of genetic testing. Nanopore sequencing technology has unique advantages in principle due to its simple sequencing principle, which does not require modification to DNA molecules and can directly sequence single molecules continuously. Compared with the first and second generation gene sequencing technologies, next-generation nanopore-based gene sequencing technology has the advantages of low cost, long read length, easy integration, portability and the like, and has been widely used in various fields.

The current sequencing technology based on biological nanopores is considered to be the most potential and disruptive among many nanopore technologies, and has been successfully commercialized. However, the current sequencing technology based on biological nanopores still faces many challenges, and one of the biggest challenges comes from the stability of the sequencing chip. In order to maintain the stability of biological nanopore proteins, it is required to embed the pore protein into a carrier membrane with a thickness of single layer of molecules. Carrier membranes are usually formed by self-assembly of amphipathic molecules, and membrane materials are usually phospholipid molecules or block polymers.

### SUMMARY

In an aspect of this disclosure, provided is a membrane forming device, including: a base; a main body arranged to the base and the main body having a recess; and an opening member arranged inside a recess port on an end of the recess away from the base, and a hollow portion of the opening member being configured to form a membrane.

In some embodiments, the recess is configured to define an accommodating chamber for accommodating a polar medium solution, the membrane is an amphipathic molecular membrane, and a cross-sectional area of the hollow portion of the opening member is smaller than that of the accommodating chamber.

In some embodiments, the main body includes: a first body layer arranged to a surface of the base; and a second body layer located on a side of the first body layer away from the base, wherein the recess penetrates the second body layer and the first body layer in a direction perpendicular to the base, and the opening member is connected to a portion of the recess penetrating the second body layer.

In some embodiments, the opening member is integrally formed with the second body layer.

In some embodiments, the opening member is integrally formed with the main body.

In some embodiments, the opening member includes: a flange protruding radially inwardly relative to the recess port, wherein an inner edge of the flange forms the hollow portion.

In some embodiments, the inner edge of the flange is located on a same plane.

In some embodiments, a cantilevered end of the flange is chamfered or rounded.

In some embodiments, a section of the flange is T-shaped or in a shape of a convex polygon.

In some embodiments, a thickness of the cantilevered end of the flange in a direction perpendicular to the base is equal to or less than a thickness of a root portion of the flange in the direction perpendicular to the base.

In some embodiments, a thickness of the flange in the direction perpendicular to the base gradually decreases from the root portion to the cantilevered end.

In some embodiments, the flange has at least one portion that protrudes or recesses from a surface of the flange in a direction perpendicular to the base.

In some embodiments, the at least one portion is at least located on a side of the flange away from the base.

In some embodiments, the inner edge of the flange has a plurality of protrusions protruding inwardly.

In some embodiments, the cross section of the hollow portion is in a shape of a circle or a convex polygon, and the cross section of the accommodating chamber is in a shape of a circle or a convex polygon.

In some embodiments, a longitudinal cross section of the accommodating chamber is in a shape of a rectangle, a trapezoid, a curved-edge trapezoid, or a drum.

In some embodiments, the membrane forming device further includes: a first electrode arranged outside the accommodating chamber; and a second electrode arranged inside the accommodating chamber.

In some embodiments, the base includes: a substrate; and at least one passivation layer, arranged between the substrate and the main body, wherein the second electrode is exposed from a bottom of the recess; and the second electrode is located between the substrate and the at least one passivation layer, or the second electrode is located on a side of the at least one passivation layer away from the substrate, or the second electrode is located between adjacent passivation layers in a case where the at least one passivation layer is configured as a plurality of passivation layers.

In some embodiments, the main body includes a plurality of recesses, and the plurality of recesses of the main body are arranged in a rectangular array, a parallelogram array, a honeycomb array, or irregularly.

In some embodiments, the opening member has a plurality of hollow portions.

In some embodiments, the membrane forming device further includes: a sealing cover plate configured to be assembled with the main body.

In some embodiments, the membrane forming device further includes: a sealing cover plate configured to be assembled with the main body; an amphipathic molecular membrane formed in the hollow portion of the opening member; a first polar medium solution accommodated in the accommodating chamber, and a second polar medium solution accommodated outside the accommodating chamber and located above the main body, the opening member and the amphipathic molecular membrane.

In some embodiments, the amphipathic molecular membrane is a monolayer lipid membrane, a bilayer lipid membrane or a high molecular polymer membrane.

In some embodiments, the membrane forming device further includes: a membrane channel embedded into the amphipathic molecular membrane.

In an aspect of this disclosure, provided is a membrane forming method, including: providing the membrane forming device according to any one of the preceding claims, and assembling a sealing cover plate to an upper side of a main body to form a flow channel between the sealing cover plate and the main body; introducing a first polar medium solution into the flow channel so that the first polar medium solution flows into a recess of the main body from a hollow portion of a opening member and fills the recess of the main body; and removing the first polar medium solution between the opening member and the sealing cover plate, and causing a non-polar medium solution and a second polar medium solution to cover the opening member, wherein at least one of the non-polar medium solution and the second polar medium solution contains amphipathic molecules to form an amphipathic molecular membrane in the hollow portion.

In some embodiments, the step of removing the first polar medium solution between the opening member and the sealing cover plate includes: introducing a gas medium into the flow channel so as to remove the first polar medium solution between the main body and the sealing cover plate and between the opening member and the sealing cover plate through the gas medium.

In some embodiments, the step of causing the non-polar medium solution and the second polar medium solution to cover the opening member includes: introducing the non-polar medium solution into the flow channel so that the non-polar medium solution covers the opening member; and introducing the second polar medium solution into the flow channel so that the second polar medium solution covers the opening member.

In some embodiments, the step of causing the non-polar medium solution and the second polar medium solution to cover the opening member includes: removing the sealing cover plate; coating the non-polar medium solution to the main body and the opening member and causing the non-polar medium solution to cover the opening member; assembling the sealing cover plate to the upper side of the main body to form a flow channel between the sealing cover plate and the main body; and introducing the second polar medium solution into the flow channel so that the second polar medium solution covers the opening member.

In some embodiments, the step of removing the first polar medium solution between the opening member and the sealing cover plate, and causing the non-polar medium solution and the second polar medium solution to cover the opening member includes: introducing the non-polar medium solution into the flow channel so as to remove a first polar medium solution between the main body and the sealing cover plate and a first polar medium solution between the opening member and the sealing cover plate by virtue of the non-polar medium solution, and to achieve coverage of the opening member by the non-polar medium solution; and introducing the second polar medium solution into the flow channel so that the second polar medium solution covers the opening member.

In some embodiments, the membrane forming method further includes: embedding a membrane channel into the amphipathic molecular membrane.

### BRIEF DESCRIPTION OF THE DRAWINGS

The accompanying drawings, which form part of the description, illustrate embodiments of the present disclosure and together with the description, serve to explain the principles of the present disclosure.

The present disclosure can be more clearly understood from the following detailed descriptions with reference to the accompanying drawings, in which:
Fig. 1 is a schematic partially cutaway three-dimensional structural diagram of a membrane forming device according to some embodiments of the present disclosure;
Figs. 2-5 are schematic longitudinal-section diagrams of the membrane forming device according to some embodiments of the present disclosure;
Fig. 6A is a schematic structural diagram of the membrane forming device according to some embodiments of the present disclosure assembled with a sealing cover plate;
Fig. 6B is a schematic diagram of the structure of the membrane forming device including a sealing cover plate, a first polar medium solution, an amphipathic molecular membrane embedded with a membrane channel, and a second polar medium solution according to some embodiments of the present disclosure;
Fig. 7 is a schematic structural diagram of electrode arrangement inside a recess of the membrane forming device according to some embodiments of the present disclosure;
(a)-(g) of Fig. 8 respectively show cross-sectional shapes of a hollow portion and a accommodating chamber in some embodiments of the membrane forming device according to the present disclosure;
(a)-(i) of Fig. 9 respectively show the shape of a flange of an opening member in some embodiments of the membrane forming device according to the present disclosure;
(a)-(d) of Fig. 10 respectively show shape of the longitudinal cross section of a accommodating chamber in some embodiments of the membrane forming device according to the present disclosure;
(a)-(c) of Fig. 11 respectively show the arrangement of a plurality of recesses of a main body in some embodiments of the membrane forming device according to the present disclosure;
(a)-(c) of Fig. 12 are schematic structural diagrams showing the open member with a plurality of hollow portions in some embodiments of the membrane forming device according to the present disclosure;

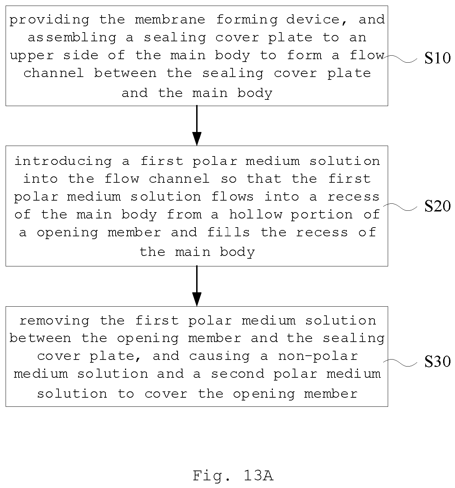

Fig. 13A is a schematic flowchart of some embodiments of a membrane forming method according to the present disclosure;

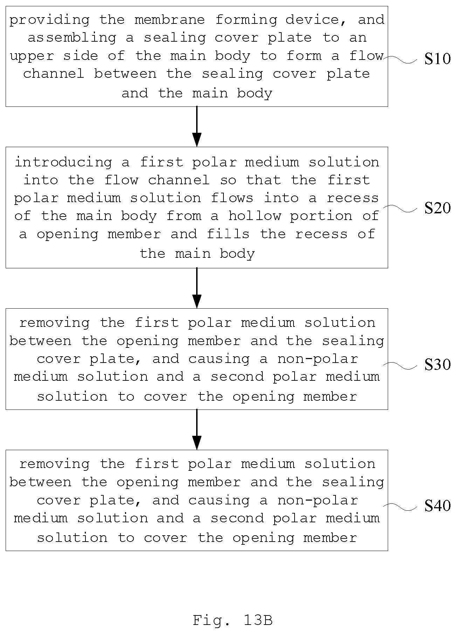

Fig. 13B is a schematic flowchart of other embodiments of the membrane forming method according to the present disclosure;

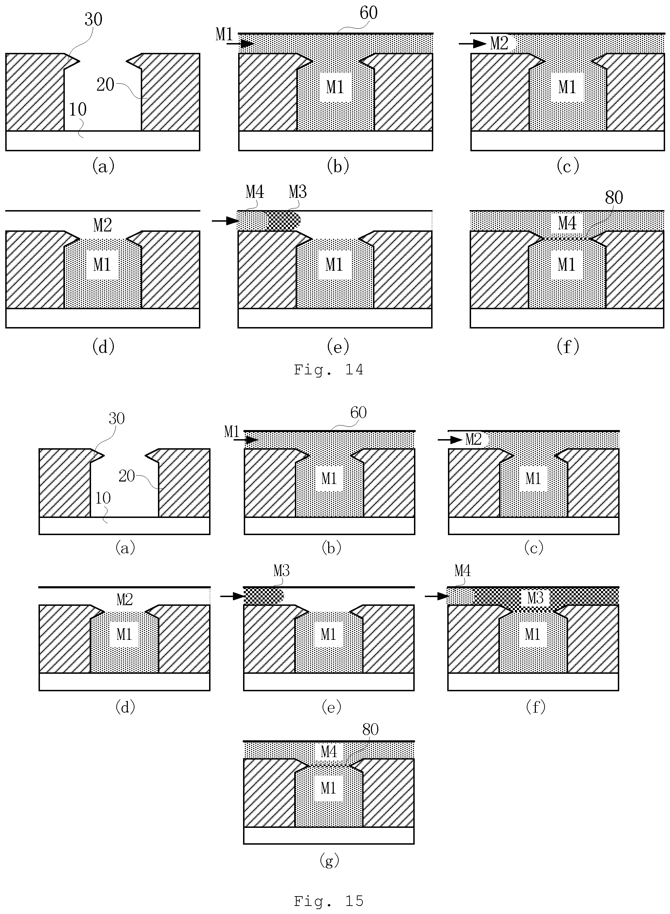

Figs. 14-17 are partially cross-sectional diagrams of the membrane forming device in a series of steps of some embodiments of the membrane forming method according to the present disclosure;

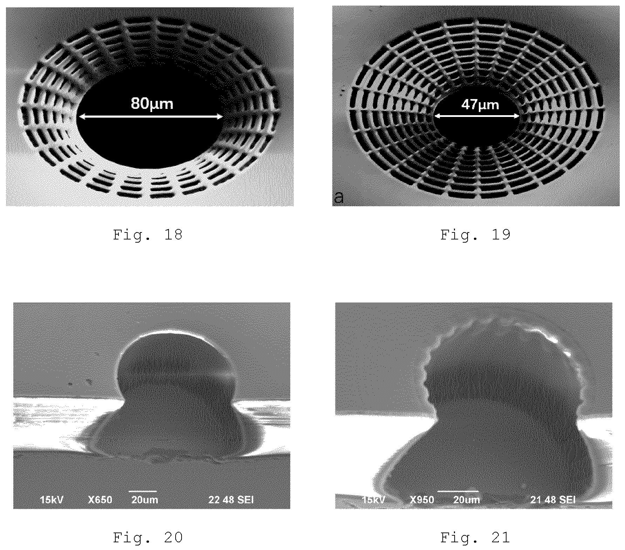

Fig. 18 is a scanning electron microscope phenogram of a first embodiment of the membrane forming device according to the present disclosure;
Fig. 19 is a scanning electron microscope phenogram of a second embodiment of the membrane forming device according to the present disclosure;
Fig. 20 is a partially cutaway scanning electron microscope phenogram of a third embodiment of the membrane forming device according to the present disclosure;
Fig. 21 is a partially cutaway scanning electron microscope phenogram of a fourth embodiment of the membrane forming device according to the present disclosure;

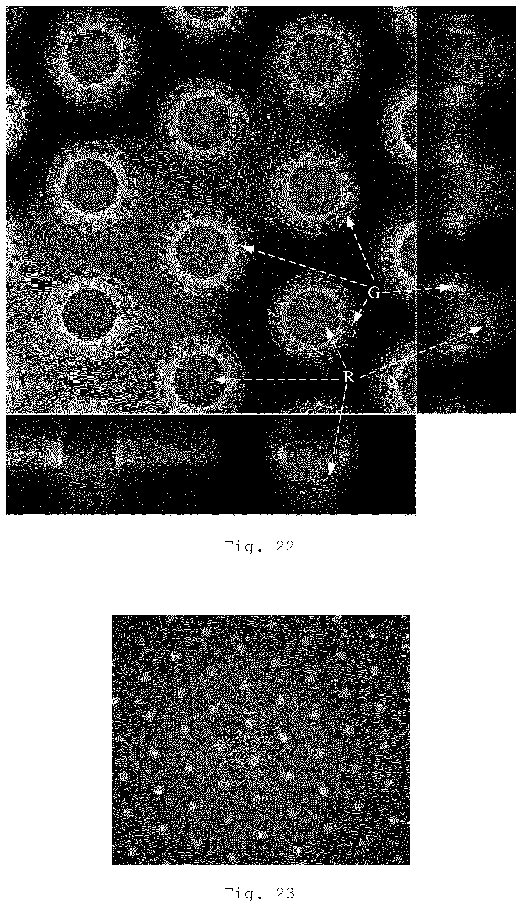

Fig. 22 is a confocal fluorescence microscopy phenogram of a membrane array in a first embodiment of the membrane forming method according to the present disclosure, taken from a top view and in a cutaway state;
Fig. 23 is a confocal fluorescence microscopy phenogram of a membrane array in a second embodiment of the membrane forming method according to the present disclosure;

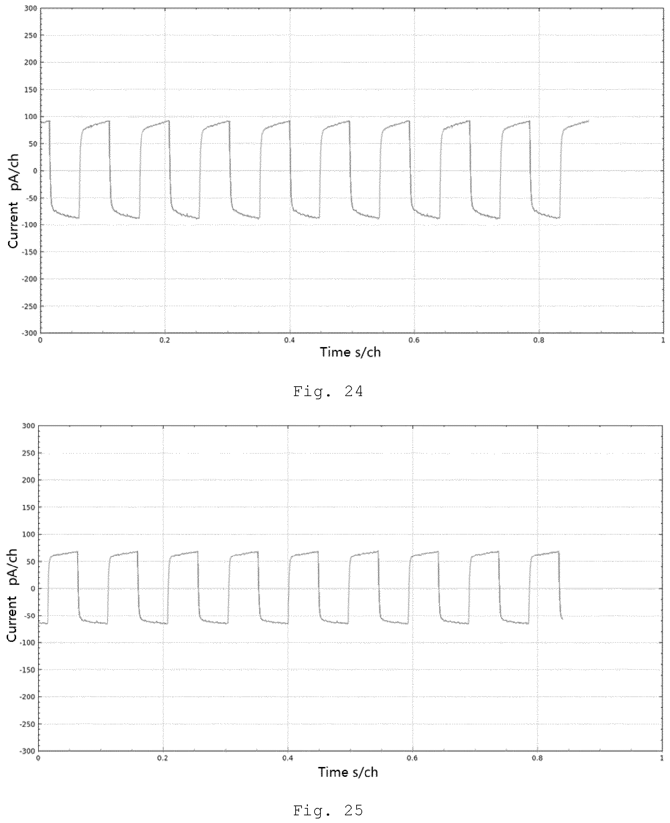

Figs. 24 and 25 are respectively graphs showing transmembrane current curves monitored when triangular wave voltage is applied to different channels after membrane formation in embodiments of the membrane forming device according to the present disclosure;

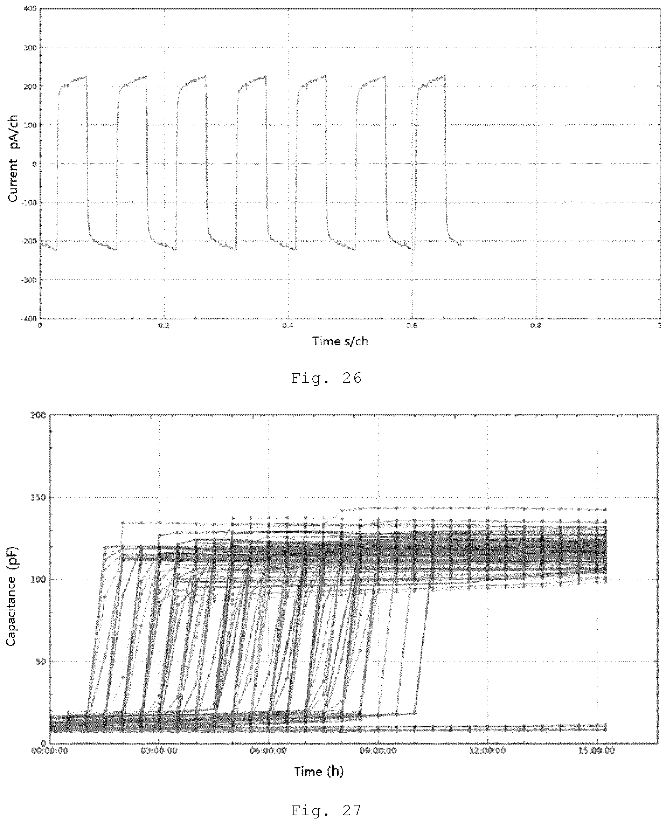

Fig. 26 is a graph showing a transmembrane current curve monitored when triangular wave voltage is applied to after membrane formation in an embodiment of the membrane forming device according to the present disclosure;
Fig. 27 is a graph showing the capacitance of multiple channels changing with time during the membrane forming process in an embodiment of the membrane forming device according to the present disclosure;

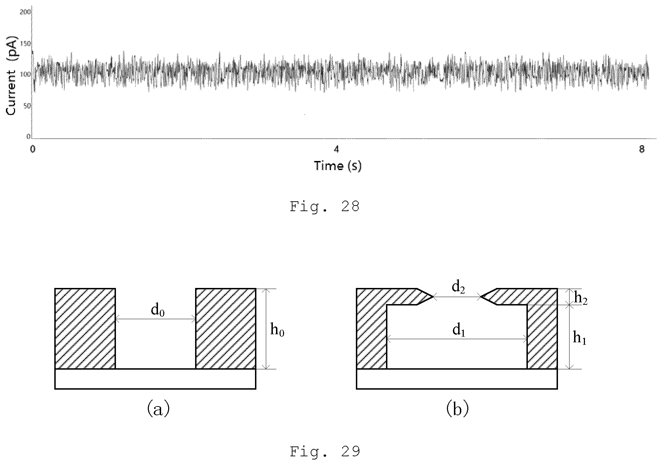

Fig. 28 is a schematic diagram of detection current signals when single molecules are detected by an electrical method using a carrier membrane formed by an embodiment of the membrane forming device according to the present disclosure;
(a) and (b) of fig. 29 are dimensioned schematic diagrams of a membrane forming device in the related art and an embodiment of the membrane forming device according to the present disclosure.

It should be understood that the dimensions of various parts shown in the accompanying drawings are not drawn according to an actual scale relationship. In addition, the same or similar reference signs indicate the same or similar components.

### DETAILED DESCRIPTION

Various exemplary embodiments of the present disclosure will now be described in detail with reference to the accompanying drawings. The descriptions of the exemplary embodiments are merely illustrative and are in no way intended to limit the present disclosure, and application or uses thereof. The present disclosure can be implemented in many different forms and is not limited to the embodiments described herein. These embodiments are provided, so that the present disclosure will be thorough and complete, and will fully convey the scope of the present disclosure to those skilled in the art. It should be noted that unless otherwise specified, the relative arrangement of components and steps, the composition of materials, numerical expressions and numerical values set forth in these embodiments should be interpreted as merely illustrative and not as limitative.

Similar words of "first", "second" and the like used in the present disclosure do not indicate any order, quantity or importance, but are only used to distinguish different parts. Similar words such as "including" or "comprising" mean that the elements before the word cover the elements listed after the word, without excluding the possibility of covering other elements. "Upper", "lower", "left", "right" and the like are only used to indicate a relative positional relationship. After the absolute position of a described object changes, the relative positional relationship may also change accordingly.

In the present disclosure, when it is described that a specific device is located between a first device and a second device, there may be an intervening device between the specific device and the first device or the second device or not. When it is described that the specific device is connected to other devices, the specific device may be directly connected to the other devices without the intervening device, and may also have the intervening device without being directly connected to the other devices.

All terms (including technical terms or scientific terms) used in the present disclosure have the same meanings as those understood by ordinary skilled in the art to which the present disclosure belongs, unless otherwise defined particularly. It should also be understood that the terms defined in, for example, general dictionaries should be interpreted as having the meanings consistent with their meanings in the context of the related art, and should not be interpreted in an idealized or extremely formal sense unless explicitly defined herein.

Technologies, methods and equipment known to those skilled in the related art may not be discussed in detail, but the technologies, methods and equipment should be regarded as part of the description under appropriate circumstances.

It is found through research by inventors that in a sequencing chip, the carrier membrane is supported by a microwell structure, and the shape and opening diameter of the support structure will directly affect the stability of the membrane. The stability of the membrane directly affects the stability of the pore protein, and the membrane formation yield directly determines the number of single nanopores. Therefore, the membrane forming device and its membrane forming method and efficiency will ultimately affect the data output and data quality of the entire sequencing system.

In some related arts, the sequencing chip contains a recess structure, and the bottom of the recess is provided with chip electrodes. The membrane forming process of the sequencing chip includes: completely covering the entire surface of the recess with a hydrophobic layer; introducing a polar medium solution, repelling the hydrophobic layer to the side wall of the recess by using the hydrophilicity of the chip electrodes so that the chip electrodes are electrically connected to the polar medium solution; discharging the polar medium solution that is outside the recess; and sequentially introducing amphipathic molecules used for the formation of the membrane and the polar medium solution to form, at the recess port of the recess, a carrier membrane composed of monolayer/bilayer amphipathic molecules.

After research, these related arts have the following shortcomings:
1. The membrane formation yield of the chip array is low, and the stability of the carrier membrane formed is poor.
2. Based on process requirements, the chip body needs to be pretreated with hydrophobic coating at first, which makes the chip processing process more complicated.
3. The membrane forming area is large and high membrane capacitance is produced, resulting in higher electrical noise, and easier embedding of multiple nanopore proteins.
4. A less volume of electrochemical solution can be accommodated in the recess, which leads to a short detection life.

In view of this, the present disclosure provides embodiments of a membrane forming device and a membrane forming method, which can improve the stability of the carrier membrane formed.

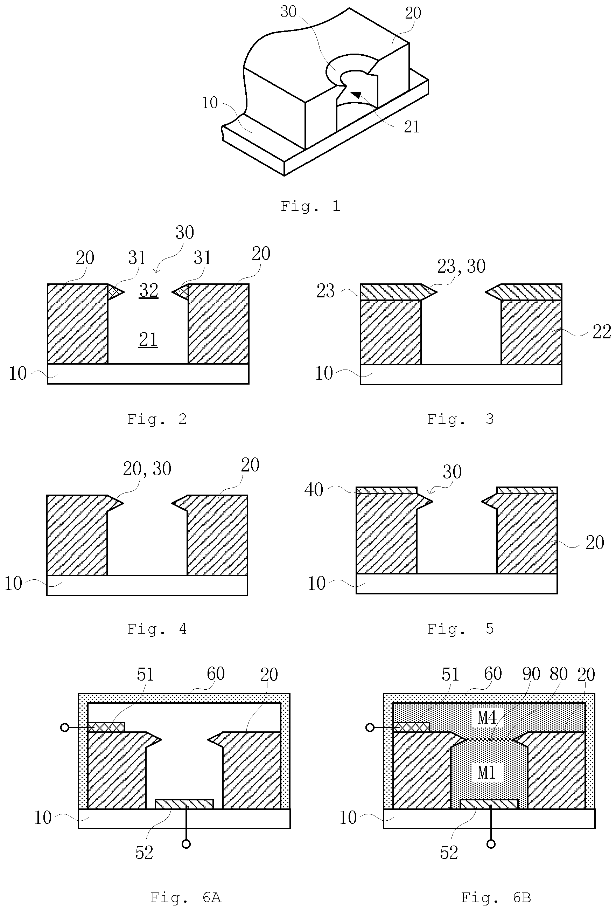

Fig. 1 is a schematic partially cutaway three-dimensional structural diagram of a membrane forming device according to some embodiments of the present disclosure. Figs. 2-5 are schematic longitudinal-section diagrams of the membrane forming device according to some embodiments of the present disclosure. Referring Figs. 1-5, in some embodiment, the membrane forming device includes: a base 10, a main body 20 and an opening member 30. The main body 20 is provided to the base 10 and has a recess. The opening member 30 is provided inside a recess port on a side of the recess away from the base 10, and a hollow portion 32 of the opening member 30 is configured to form a membrane 80.

In some embodiments, the recess defines an accommodating chamber 21 for accommodating a polar medium solution, the cross-sectional area of the hollow portion 32 of the opening member 30 is smaller than that of the accommodating chamber 21, and the membrane 80 is an amphipathic molecular membrane.

In a case where the membrane forming device of this embodiment is used to form a carrier membrane composed of amphipathic molecules (i.e., an amphipathic molecular membrane), since the formation of the carrier membrane is a dynamically stable self-assembly process, the shape and size of the final stabilized carrier membrane are determined by the shape and size of the hollow portion of the opening member. By virtue of the hollow portion of the opening member with a smaller cross-sectional area, the area of the carrier membrane formed can be effectively reduced, thereby improving the stability of the carrier membrane and further improving the mechanical stability of the overall chip. In addition, reducing the area of the carrier membrane can effectively reduce the membrane capacitance of the carrier membrane, thereby reducing the electrical noise of the system. By virtue of a accommodating chamber with a relatively large cross-sectional area, the volume of the electrochemical solution in the accommodating chamber and the detection life of the device can be increased.

Embodiments of the membrane forming device according to the present disclosure can be applied to the field of medical devices for in vitro diagnostic testing such as gene sequencing, protein sequencing, and biomolecule testing. For example, nanopore proteins are embedded in the formed carrier membrane to test nucleic acid molecules. Embodiments of the membrane forming device according to the present disclosure may also be applied to high-end manufacturing or semiconductor fields such as MEMS sensors and biosensors, or to scientific research fields such as life science research and brain/neuroscience research.

In Fig. 1, the accommodating chamber 21 can accommodate a liquid-state polar medium solution. The polar medium solution can flow into the accommodating chamber 21 from the upper side of the main body 20 through the opening member 30. The polar medium solution may be an electrochemical solution, such as an aqueous solution of potassium chloride or a potassium ferricyanide/potassium ferrocyanide solution. The material of the main body 20 may be non-conductive photoresist or other non-conductive materials. The material of the main body 20 may also include semiconductor materials or conductive materials, and a non-conductive passivation layer is formed to surfaces of the semiconductor materials or the conductive materials.

The base 10 may be configured as a single-layer or multi-layer structure. The main body 20 may be configured as a single-layer or multi-layer structure. The opening member 30 may be integrally formed with the whole or a part of the main body 20, or may be formed separately from the main body 20 and connected to the main body 20. The base 10 may provide support and forming surface for other parts of the membrane forming device. For the main body 20 including a plurality of recesses, the base 10 and the main body 20 may select materials that can achieve electrical insulation to achieve electrical insulation between adjacent electrodes.

Referring to Fig. 2, in some embodiments, the main body 20 is configured as a single-layer structure, and the opening member 30 is connected to the inner side of the recess port of the recess of the main body 20. The opening member 30 may be formed after the formation of the main body. For example, the main body 20 may be formed by performing a single photolithography step.

Referring to Fig. 3, in some embodiments, the main body 20 includes: a first body layer 22 and a second body layer 23. The first body layer 22 is provided to a surface of the base 10. The second body layer 23 is located on a side of the first body layer 22 away from the base 10. The recess penetrates the second body layer 23 and the first body layer 22 in a direction perpendicular to the base 10, and the opening member 30 is connected to a portion of the recess penetrating the second body layer 23.

The first body layer 22 may be configured as a single-layer structure or a multi-layer structure. The opening member 30 may be integrally formed with the second body layer 23 to simplify the process. The first body layer 22 and the second body layer 23 may be formed by stacking through photolithography many times or bonding processes. The first body layer 22 and the second body layer 23 may be made of the same material or different materials. Materials that may be used for all layers of the main body include but are not limited to: silicon-based materials, photoresist, etc. For example, in a stacked main body 20, the first body layer 22 is made of a silicon-based material, and the second body layer 23 is made of a photosensitive epoxy resin photoresist.

Referring to Fig. 4, in some embodiments, the main body 20 is configured as a single-layer structure, and the opening member 30 is integrally formed with the main body 20 to simplify the process. The main body 20 and the opening member 30 may be formed of the same material, or may be formed of different materials.

Referring to Fig. 5, in some embodiments, a one-layer or multi-layer layered structure 40, such as an electrode layer, may be provided on the side of the main body 20 away from the substrate 10 to implement set functions.

In Fig. 2, the opening member 30 may include: a flange 31 protruding radially inwardly relative to the recess port. The inner edge of the flange 31 forms the hollow portion 32. The hollow portion 32 can be used to form an amphipathic molecular membrane to allow insertion of membrane channels. Membrane channels may be nanopores, naturally occurring pores, mutant pores derived from naturally occurring pores, or synthetic pores. The pores can be homo-oligomeric (derived from the same monomer); or hetero-oligomeric (where at least one monomer is different from the others).

Referring to Figs. 1-5, in some embodiments, the inner edge of the flange 31 is located on a same plane to facilitate processing and help form a more stable carrier membrane. In other embodiments, the inner edge of the flange 31 may not be located on a same plane.

Fig. 6A is a schematic structural diagram of the membrane forming device according to some embodiments of the present disclosure assembled with a sealing cover plate. Referring to Fig. 6A, in some embodiments, the membrane forming device further includes: a first electrode 51 arranged outside the accommodating chamber 21 and a second electrode 52 arranged inside the accommodating chamber 21. The first electrode 51 may be implemented by a conductive layer deposited to the surface of one side of the main body 20 away from the base 10. The second electrode 52 may be arranged to the bottom of the accommodating chamber 21, or may be arranged to a side wall of the accommodating chamber 21, or suspended in the accommodating chamber 21. The second electrode 52 may also be embedded between a plurality of body layers included in the main body.

The first electrode 51 and the second electrode 52 are electrically connected to the aqueous solution outside and inside the accommodating chamber 21 respectively, so that electrical signals across the amphipathic molecular membrane can be measured through the connection of a circuit to the first electrode 51 and the second electrode 52. The circuit can adopt the same structure as a conventional circuit that detects electrical signals across the amphipathic molecule layer.

The first electrode 51 and the second electrode 52 are electrochemical electrodes in contact with the aqueous solution and capable of measuring lower current, and have stability relative to the aqueous solution. The materials that may be used for the first electrode 51 include but are not limited to: silver/silver chloride. Materials that may be used for the second electrode 52 include but are not limited to: silver/silver chloride, precious metals such as gold, platinum, and palladium, or conductor such as graphene or titanium nitride, or semiconductor materials. The materials used for the first electrode 51 and the second electrode 52 may be the same or different. The first electrode 51 and the second electrode 52 may also be composite electrodes formed by stacking or mixing multiple materials. The surfaces of the first electrode 51 and the second electrode 52 may be covered or not covered with materials such as conductive polymers and hydrogels.

In other embodiments, the membrane forming device may not include electrodes. Correspondingly, other methods, such as optical signal detection, may be used to achieve applications such as DNA sequencing, protein analysis, single cell analysis, or drug screening.

In order to facilitate the formation of the amphipathic molecular membrane, referring to Fig. 6A, in some embodiments, the membrane forming device further includes a sealing cover plate 60. In the membrane forming device, the sealing cover plate 60 is detachable. The sealing cover plate 60 can be assembled with the main body 20 to form a flow channel located above the main body 20, and the flow channel may be connected to an inlet and an outlet (not shown) formed in the sealing cover plate 60. The operator can introduce a gas medium, a polar medium solution or non-polar medium solution into the flow channel through the inlet during the formation of the amphipathic molecular membrane.

Fig. 6B is a schematic diagram of the structure of the membrane forming device including a sealing cover plate, a first polar medium solution, an amphipathic molecular membrane embedded with a membrane channel, and a second polar medium solution according to some embodiments of the present disclosure. Referring to Fig. 6B, in some embodiments, the membrane forming device further includes: a sealing cover plate 60, an amphipathic molecular membrane 80, a first polar medium solution M1, and a second polar medium solution M4. The sealing cover plate 60 is configured to be assembled with the main body 20 to form a flow channel located above the main body 20, and the flow channel may be connected to an inlet and an outlet (not shown) formed in the sealing cover plate 60.

The amphipathic molecular membrane 80 is formed in the hollow portion 32 of the opening member 30. The first polar medium solution M1 is accommodated in the accommodating chamber 21, the second polar medium solution M4 is accommodated outside the accommodating chamber 21 and located above the main body 20, the opening member 30 and the amphipathic molecular membrane 80.

The amphipathic molecular membrane 80 may be formed in the hollow portion 32, but is not limited to be formed in the hollow portion 32, and may also extend to the upper side and/or lower side of the flange 31. The amphipathic molecular membrane 80 may be located on an interface between the first polar medium solution M1 and the second polar medium solution M4, and part of a non-polar medium solution M3 may remain in the layer where the amphipathic molecular membrane 80 is located. The amphipathic molecular membrane 80 may be directly attached to the flange 31, or may be maintained in the hollow portion 32 through the attachment of the non-polar solution M3 to the flange 31.

In this embodiment, the first polar medium solution M1 and the second polar medium solution M4 may be the same or different liquids, usually a conductive salt solution, such as an aqueous solution of potassium chloride, or potassium ferricyanide/potassium ferrocyanide solution or the like. The amphipathic molecular membrane 80 may be a monolayer lipid (e.g., phospholipid monolayer), a bilayer lipid (e.g., phospholipid bilayer) or a polymer membrane (e.g., di-block, tri-block).

Referring to Fig. 6B, in some embodiments, membrane channels are embedded into the amphipathic molecular membrane. Optionally, the membrane channel is, for example, a nanopore protein. The nanopore protein refers to a protein that can form a transmembrane protein pore. The transmembrane protein pore is usually formed by multiple (such as 6, 7 or 8) repeating subunits. These subunits generally surround a central axis and provide a channel that allows ions to flow therein. Transmembrane protein pores allow ions to flow down their electrochemical gradient from one side of the membrane to the other side .

In some embodiments, the nanopore protein is chose from: hemolysin, MspA, Frac, ClyA, PA63, CsgG, GspD, XcpQ, Wza, SP1, Phi29 connector, SPP1 connector, T3 connector, T4 connector, T7 connector, K ion channel protein, Na ion channel protein and Ca ion channel protein.

Membrane channels may be nanopores, naturally occurring pores, mutant pores derived from naturally occurring pores, or synthetic pores. The pores can be homo-oligomeric (derived from the same monomer); or hetero-oligomeric (where at least one monomer is different from the others).

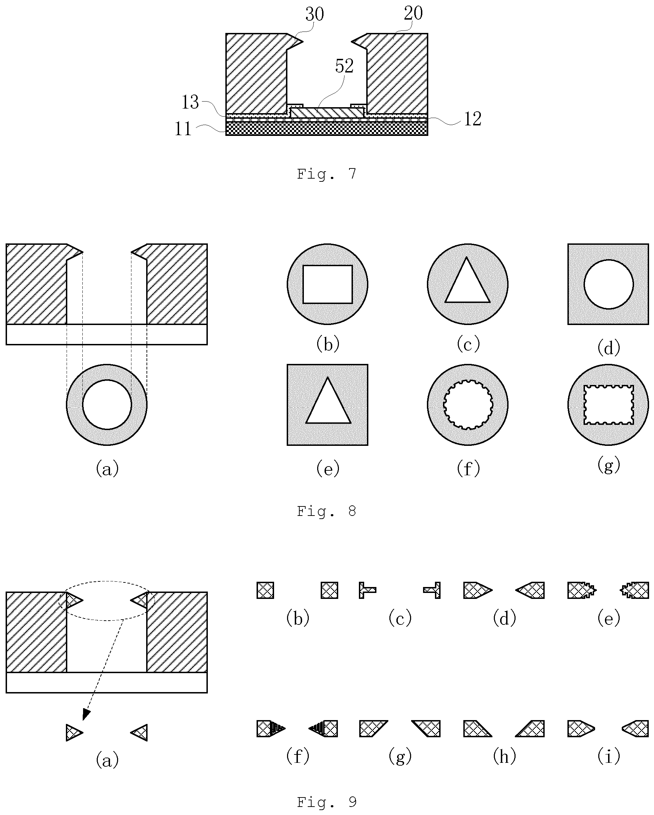

Fig. 7 is a schematic structural diagram of electrode arrangement inside a recess of the membrane forming device according to some embodiments of the present disclosure. Referring to Fig. 7, in some embodiments, the base 10 includes: a substrate 11 and at least one passivation layer (e.g., passivation layers 12 and 13) . The at least one passivation layer is arranged between the substrate 11 and the main body 20. The second electrode 52 is exposed from a bottom of the recess so as to be in contact with the aqueous solution in the accommodating chamber 21.

The second electrode 52 may be located between the substrate 11 and the at least one passivation layer, or may be located on a side of the at least one passivation layer away from the substrate 11. In a case where at least one passivation layer is configured as a plurality of passivation layers, for example, the two passivation layers 12 and 13 shown in Fig. 7, and the second electrode 52 may be located between adjacent passivation layers 12 and 13.

The substrate 11 may be a silicon-based substrate, a glass substrate, a carbon-based substrate, an integrated circuit wafer, a printed circuit board, a flexible circuit board, etc. The material of the passivation layer may be a conventional silicon-based passivation material such as silicon dioxide and silicon nitride, or a thin layer of photoresist, such as epoxy resin photoresist or polyimide film, etc., or it may also be an insulating paint commonly used for passivating printed circuit boards, such as acrylic resin, etc.

(a)-(g) of Fig. 8 respectively show cross-sectional shapes of the hollow portion and the accommodating chamber in some embodiments of the membrane forming device according to the present disclosure; Referring to Fig. 8, the orthographic projection of the cross section of the hollow portion 32 on the base is located within the cross section of the accommodating chamber. The cross section of the hollow portion 32 and the cross section of the accommodating chamber 21 may be the same or different in shape, wherein the cross section of the hollow portion 32 may be in the shape of a circle or a convex polygon, and the cross section of the accommodating chamber 21 may be in the shape of a circle or a convex polygon. The convex polygon may be in the shape of a triangle, a rectangle, a square, a hexagon, an octagon, or the like. The hollow portion with a circular or convex polygonal cross section is more likely to form a stable carrier membrane. The accommodating chamber with a circular or convex polygonal cross section can accommodate a large volume of aqueous solution.

In (a)-(c) of Fig. 8, the cross sections of the accommodating chambers 21 are all circles. In (d) and (e) of Fig. 8, the cross sections of the accommodating chambers 21 are all squares. In (a) and (d) of Fig. 8, the cross sections of the hollow portions 32 are both circles. In (b), (c) and (e) of Fig. 8, the cross sections of the hollow portion 32 are a rectangle and triangles respectively.

In order to improve the adhesion of the hollow portion 32 to the carrier membrane, referring to (f) and (g) of Fig. 8, in some embodiments, the inner edge of the flange 31 have a plurality of protrusions protruding inwardly. The uneven edge microstructure formed by the plurality of protrusions can form a carrier membrane with higher stability.

(a)-(i) of Fig. 9 respectively show the shape of the flange of the opening member in some embodiments of the membrane forming device according to the present disclosure. Referring to Fig. 9, in some embodiments, the section of the flange 31 may be T-shaped or in the shape of a convex polygon. In (c) of Fig. 9, the thickness, in a direction perpendicular to the base 10, of a cantilevered end of the flange 31 having a T-shaped section is equal to or less than the thickness of a root portion of the flange 31 in the direction perpendicular to the base 10. The opening member of this structure is more reliable and can form a carrier membrane with higher stability.

In (a) of Fig. 9, the cross section of the flange 31 is in the shape of a triangle. The thickness of the flange 31 in the direction perpendicular to the base 10 gradually decreases from the root portion to the cantilevered end. This gradual acute-angled edge flange 21 can increase the assembly stress of the carrier membrane and improve the stability of the carrier membrane.

In (b) of Fig. 9, the cross section of the flange 31 is in the shape of a rectangle. In (d) of Fig. 9, the section of the flange 31 is in the shape of a convex pentagon, which means that the root portion of the flange 31 is rectangular and the cantilevered end is triangular. In (g) to (h) of Fig. 9, the section of the flange 31 is in the shape of a convex quadrilateral, and the position of the inner edge of the cantilevered end may be flush with the highest position or the lowest position of the flange 31.

Increasing of the specific surface area of the flange 31 can make the attachment of the non-polar medium solution easier and improve the stability of the formed carrier membrane. Referring to (i) of Fig. 9, the cantilevered end of the flange 31 may also be chamfered or rounded. Referring to (e) and (f) of Fig. 9, in some embodiments, the flange 31 has at least one portion that protrudes or recesses from a surface of the flange 31 in the direction perpendicular to the base 10. The portion here may be a discretely or continuously raised or recessed microstructure. In (f) of Fig. 9, the at least one portion is at least located on a side of the flange 31 away from the base 10. The carrier membrane may not be limited to being formed in the hollow portion, but may also be formed to the upper or lower surface of the flange 31. The local microstructure helps to maintain the carrier membrane to the surface of the flange 31.

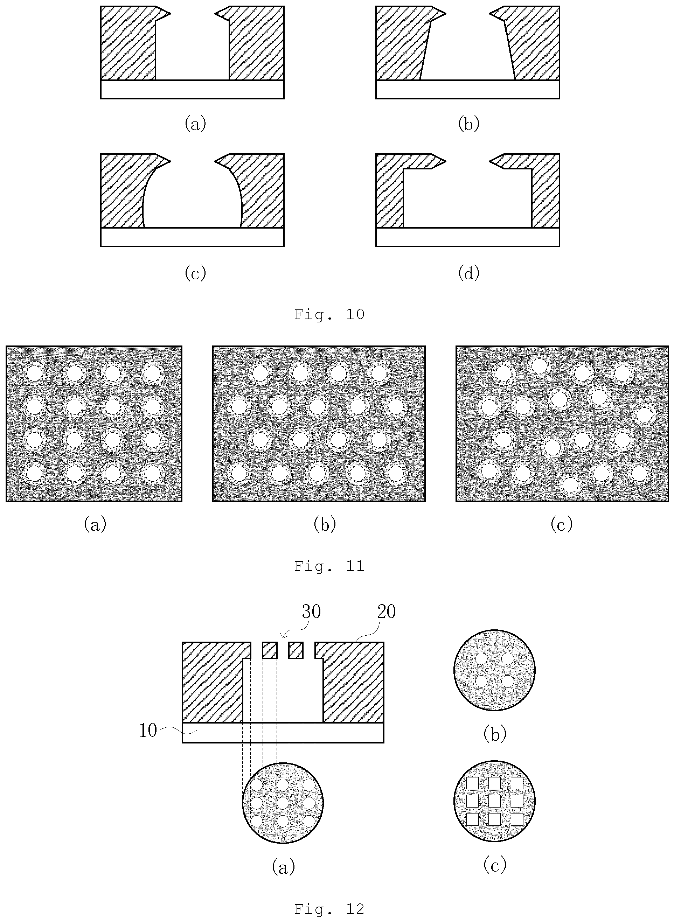

(a) - (d) of Fig. 10 respectively show shape of the longitudinal cross section of a accommodating chamber in some embodiments of the membrane forming device according to the present disclosure. Referring to Fig. 10, in some embodiments, the longitudinal cross section of the accommodating chamber 21 is in the shape of a rectangle, a trapezoid, a curved-edge trapezoid, or a drum. The side walls of the recess defining the accommodating cavity 21 may be in a vertical structure, and may also be in a bevel structure or a curved surface structure, or any combination of curved surface, bevel surface and vertical structures.

In (a) and (d) of Fig. 10, the longitudinal cross sections of the accommodating chambers 21 are both rectangles but differ in that (d) of Fig. 10 realizes a larger rectangle, thereby forming a accommodating chamber with a larger volume, and this is beneficial to increasing the detection life of the membrane forming device. In (b) and (c) of Fig. 10, the longitudinal cross sections of the accommodating chambers 21 are a trapezoid and a curved-edge trapezoid, respectively.

(a)-(c) of Fig. 11 respectively show the arrangement of a plurality of recesses of the main body in some embodiments of the membrane forming device according to the present disclosure. Referring to Fig. 11, in some embodiments, the main body 20 may include a plurality of recesses. From a top view, the plurality of recesses may be arranged to the main body 20 in various forms, such as a rectangular array, a parallelogram array, a honeycomb array, or irregularly. For example, in (a) of Fig. 11, the plurality of recesses are arranged in a rectangular array, where the recesses are arranged with substantially the same row spacing and substantially the same column spacing. In (b) of Fig. 11, the plurality of recesses are arranged in a honeycomb array. In (c) of Fig. 11, the plurality of recesses are arranged irregularly.

(a)-(c) of Fig. 12 are schematic structural diagrams showing the open member with a plurality of hollow portions in some embodiments of the membrane forming device according to the present disclosure. Referring to Fig. 12, in some embodiments, the opening member 30 has a plurality of hollow portions 32. This opening member 30 does not include a flange 31, but a plurality of through holes are formed as a plurality of hollow portions 32 in the solid opening member. The plurality of hollow portions 32 may respectively form smaller-sized carrier membranes. Depending on application requirements, membrane channels, such as nanopore proteins, may be embedded into the carrier membrane formed in one or more hollow portions 32 of the opening member 30.

In (a) and (c) of Fig. 12, the opening member 30 has a larger number of hollow portions 32, while in (b) of Fig. 12, the opening member 30 has a smaller number of hollow portions 32. The plurality of hollow portions 32 may be arranged with the same or different spacing. The cross section of each hollow portion 32 may be in the shape of a circle or a convex polygon.

Based on the above embodiments of the membrane forming device according to the present disclosure, the present disclosure further provides a corresponding membrane forming method. Fig. 13A is a schematic flowchart of some embodiments of the membrane forming method according to the present disclosure. Referring to Fig. 13A, in some embodiments, the membrane forming method includes: step S10 to step S30.

In the step S10, any embodiment of the membrane forming device described above is provided, and a sealing cover plate 60 is assembled to an upper side of a main body 20 to form a flow channel between the sealing cover plate 60 and the main body 20.

In the step S20, a first polar medium solution M1 is introduced into the flow channel so that the first polar medium solution M1 flows into a recess of the main body 20 from the hollow portion 32 of the opening member 30 and fills the recess of the main body 20.

In the step S30, the first polar medium solution M1 between the opening member 30 and the sealing cover plate 60 is removed, and the non-polar medium solution M3 and the second polar medium solution M4 are caused to cover the opening member 30.

In this embodiment, at least one of the non-polar medium solution M3 and the second polar medium solution M4 contains amphipathic molecules to form an amphipathic molecular membrane 80 in the hollow portion 32. The amphipathic molecular membrane 80 may be formed in the hollow portion 32, but is not limited to be formed in the hollow portion 32, and may also extend to the upper side and/or lower side of the flange 31. The layer where the amphipathic molecular membrane 80 is located can retain part of the non-polar medium solution M3. The amphipathic molecular membrane 80 may be directly attached to the flange 31, or may be maintained in the hollow portion 32 through the attachment of the non-polar solution M3 to the flange 31.

The amphipathic molecular membrane may be a monolayer/bilayer molecular membrane composed of amphipathic molecules. Amphipathic molecules include but are not limited to lipids, such as 1,2-bis(diphenylphosphine)ethane (DPPE) phospholipid molecules, which can also be polymers, such as di-block and tri-block. The lipids can include head groups and hydrophobic tail groups. In the formed amphipathic molecular membrane, the head group of the lipid may face the polar medium solution, while the hydrophobic tail group faces the non-polar medium solution.

The first polar medium solution M1 and the second polar medium solution M4 may be the same or different liquids, usually a conductive salt solution, such as an aqueous solution of potassium chloride, or potassium ferricyanide/potassium ferrocyanide solution or the like. The non-polar medium solution M3 may be a solvent insoluble in water, such as n-hexadecane, n-decane, n-hexane, and silicone oil.

Fig. 13B is a schematic flowchart of some embodiments of the membrane forming method according to the present disclosure. Referring to Fig. 13B, compared with Fig. 13A, in some embodiments, the membrane forming method further includes step S40. In the step S40, the membrane channel 90 is embedded into the amphipathic molecular membrane 80. Step S40 may be performed after the formation of a stable amphipathic molecular membrane.

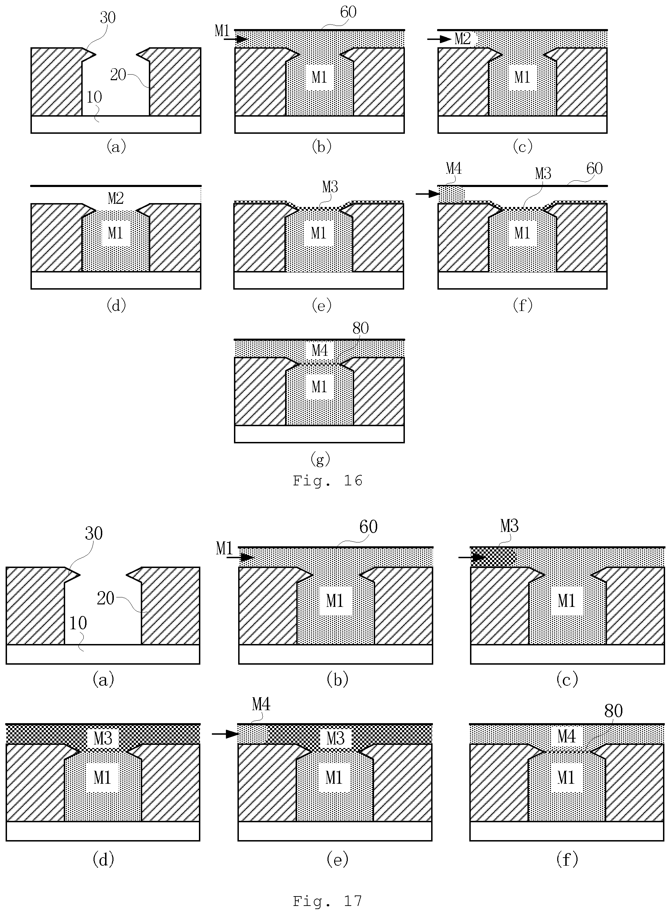

Figs. 14-17 are partially cross-sectional diagrams of the membrane forming device in a series of steps of some embodiments of the membrane forming method according to the present disclosure. Referring to (a) and (b) of Fig. 14 to (a) and (b) of Fig. 17, the assembly of the sealing cover plate 60 and the introduction of the first polar medium solution M1 are performed through steps S10 and S20, respectively. In the process of introducing the first polar medium solution M1, the first polar medium solution M1 flows into the recess of the main body 20 from the hollow portion 32 of the opening member 30 and fills the recess of the main body 20.

Referring to (c) and (d) of Fig. 14 to (c) and (d) of Fig. 16, in some embodiments, the step of removing the first polar medium solution M1 between the opening member 30 and the sealing cover plate 60 in the step S30 includes: introducing a gas medium M2 into the flow channel so as to remove the first polar medium solution M1 between the main body 20 and the sealing cover plate 60 and between the opening member 30 and the sealing cover plate 60 through the gas medium M2.

The gas medium M2 may be any one or a combination of air, nitrogen, argon, etc. The introduced gas medium M2 can discharge the first polar medium solution M1 above the main body 20 and the opening member 30, thereby forming an interface between the first polar medium solution M1 and the gas medium M2 in the hollow portion 32 of the opening member 30.

In other embodiments, the first polar medium solution M1 between the opening member 30 and the sealing cover plate 60 may also be removed by other means, such as introducing a liquid insoluble with the first polar medium solution M1, such as a non-polar medium solution.

Referring to Figs. 14 and 15, in some embodiments, the step of causing a non-polar medium solution M3 and a second polar medium solution M4 to cover the opening member 30 in the step S30 includes: introducing the non-polar medium solution M3 into the flow channel so that the non-polar medium solution M3 covers the opening member 30; and introducing the second polar medium solution M4 into the flow channel so that the second polar medium solution M4 covers the opening member 30.

In the step S30, the second polar medium solution M4 may be introduced into the flow channel immediately after the non-polar medium solution M3 is introduced into the flow channel as shown in (e) of Fig. 14, or the second polar medium solution M4 may be introduced into the flow channel waiting for a period of time after the non-polar medium solution M3 is introduced into the flow channel, as shown in (e) and (f) of Fig. 15.

The amphipathic molecules can be dissolved in the non-polar medium solution M3, or in the second polar medium solution M4, or in both the non-polar medium solution M3 and the second polar medium solution M4. After the non-polar medium solution M3 or the second polar medium solution M4 containing the amphipathic molecules is introduced, with reference to (f) of Fig. 14 and (g) of Fig. 15, a membrane 80 that separates the first polar medium solution M1 from the second polar medium solution M4 is formed in the hollow portion of the opening member 30. The membrane 80 contains amphipathic molecules that can self-assemble, and gradually thins down and expands the area through self-assembly until it reaches stability.

Referring to Fig. 16, in some embodiments, the step of causing the non-polar medium solution M3 and the second polar medium solution M4 to cover the opening member 30 in the step S30 includes: removing the sealing cover plate 60; coating the non-polar medium solution M3 to the main body 20 and the opening member 30 and causing the non-polar medium solution M3 to cover the opening member 30; assembling the sealing cover plate 60 to the upper side of the main body 20 to form a flow channel between the sealing cover plate 60 and the main body 20; and introducing the second polar medium solution M4 into the flow channel so that the second polar medium solution M4 covers the opening member 30.

In (e) of Fig. 16, the sealing cover plate 60 is removed, thereby exposing the main body 20 and the opening member 30. Then, a layer of non-polar medium solution M3 is applied as uniformly as possible onto the surfaces of the main body 20 and the opening member 30. The non-polar medium solution M3 may be applied by a dripping method, an atomized spraying method or other methods. In (f) of Fig. 16, the sealing cover plate 60 is reassembled to the upper side of the main body 20, and then the second polar medium solution M4 is introduced into the formed flow channel. The second polar medium solution M4 can discharge the non-polar medium solution M3 that is outside the opening member 30.

The amphipathic molecules can be dissolved in the non-polar medium solution M3, or in the second polar medium solution M4, or in both the non-polar medium solution M3 and the second polar medium solution M4. Referring to (g) of Fig. 16, the membrane 80 that separates the first polar medium solution M1 from the second polar medium solution M4 is formed in the hollow portion of the opening member 30. The membrane 80 contains amphipathic molecules that can self-assemble, and gradually thins down and expands the area through self-assembly until it reaches stability.

Referring to Fig. 17, in some embodiments, the step of removing the first polar medium solution M1 between the opening member 30 and the sealing cover plate 60 and causing the non-polar medium solution M3 and the second polar medium solution M4 to cover the opening member 30 in the step S30 includes: introducing the non-polar medium solution M3 into the flow channel so as to remove the first polar medium solution M1 between the main body 20 and the sealing cover plate 60 and the first polar medium solution M1 between the opening member 30 and the sealing cover plate 60 by virtue of the non-polar medium solution M3, and to achieve coverage of the opening member 30 by the non-polar medium solution M3; and introducing the second polar medium solution M4 into the flow channel so that the second polar medium solution M4 covers the opening member 30.

In (c) of Fig. 17, the non-polar medium solution M3 is introduced into the flow channel. At this time, under the action of shear force, an interface between the first polar medium solution M1 and the non-polar medium solution M3, as shown in (d) of Fig. 17 is formed in the hollow portion of the opening member 30. In (e) of Fig. 17, the second polar medium solution M4 is introduced into the flow channel. The amphipathic molecules can be dissolved in the non-polar medium solution M3, or in the second polar medium solution M4, or in both the non-polar medium solution M3 and the second polar medium solution M4. After the non-polar medium solution M3 or the second polar medium solution M4 containing the amphipathic molecules is introduced, with reference to (f) of Fig. 17, the membrane 80 that separates the first polar medium solution M1 from the second polar medium solution M4 is formed in the hollow portion of the opening member 30. The membrane 80 contains amphipathic molecules that can self-assemble, and gradually thins down and expands the area through self-assembly until it reaches stability.

Compared with the related art, various embodiments of the membrane forming method described above in the present disclosure do not require hydrophobic pretreatment of the base, the main body, the opening member, etc. of the membrane forming device, which reduces the preparation difficulty of the membrane forming device. Based on the opening member of the membrane forming device, the shear uniformity of the interface between the gas medium or the non-polar medium solution and the first polar medium solution during the membrane forming process is optimized, so that the amphipathic molecular membrane can be completed in one go, which simplifies the membrane forming process, improves membrane formation yield and membrane stability.

The effects of the embodiments of the membrane forming device and the membrane forming method will be illustrated below from multiple aspects on the basis of experimental data.

Fig. 18 is a scanning electron microscope phenogram of a first embodiment of the membrane forming device according to the present disclosure. Referring to Fig. 18, in the first embodiment of the membrane forming device, the diameter of the hollow portion of the opening member of the membrane forming device is about 80 µm. Multiple sets of micro-groove structures arranged circumferentially are provided to the flange of the opening member, and each set of micro-groove structures includes multiple micro-groove structures arranged radially. This micro-groove structure is beneficial to the formation of the interface between the gas medium or non-polar medium solution and the first polar medium solution and the maintenance of the carrier membrane. The structure is formed by spin-coating SU-8 photoresist (with a final thickness of 95 µm) on a silicon substrate, and then carrying out processes such as pre-baking, exposure, post-baking, development, and hardbaking.

Fig. 19 is a scanning electron microscope phenogram of a second embodiment of the membrane forming device according to the present disclosure. Referring to Fig. 19, in the second embodiment of the membrane forming device, the diameter of the hollow portion of the opening member of the membrane forming device is less than 50 µm, and measured to be approximately 47 µm as final. Multiple sets of micro-groove structures arranged circumferentially are provided to the flange of the opening member, and each set of micro-groove structures includes multiple micro-groove structures arranged radially. This micro-groove structure is beneficial to the formation of the interface between the gas medium or non-polar medium solution and the first polar medium solution and the maintenance of the carrier membrane. The structure is formed by spin-coating SU-8 photoresist (with a final thickness of 95 µm) on a silicon substrate, and then carrying out processes such as pre-baking, exposure, post-baking, development, and membrane hardening.

Fig. 20 is a partially cutaway scanning electron microscope phenogram of a third embodiment of the membrane forming device according to the present disclosure. Referring to Fig. 20, in the third embodiment of the membrane forming device, the diameter of the hollow portion of the opening member of the membrane forming device is about 80 µm. The shape of the hollow portion of the opening member is approximately circular, and the flange is smooth without microstructure. The side wall of the accommodating chamber inside the opening member is a smoothly curved surface.

Fig. 21 is a partially cutaway scanning electron microscope phenogram of a fourth embodiment of the membrane forming device according to the present disclosure. Referring to Fig. 21, in the fourth embodiment of the membrane forming device, the diameter of the hollow portion of the opening member of the membrane forming device is about 75 µm. The shape of the hollow portion of the opening member is an irregular pattern, generally circular, and its inner edge has a plurality of about 3 µm raised microstructures protruding inwardly. This raised microstructure can enhance the stability of the carrier membrane. The side wall of the accommodating chamber inside the opening member is a smoothly curved surface.

Fig. 22 is a confocal fluorescence microscopy phenogram of a membrane array in a first embodiment of the membrane forming method according to the present disclosure, taken from a top view and in a cutaway state. Referring to Fig. 14, in the first embodiment of the membrane forming method, an aqueous solution of potassium chloride (500 mM) and Hepes (5mM), as the first polar medium solution, is introduced into the flow channel of the membrane forming device including a plurality of recesses ((b) of Fig. 11) and the aqueous solution fills the accommodating chamber. Then, 10 mL of air is pushed in from the flow channel of the membrane forming device to completely discharge the aqueous solution directly above the main body and the opening member, while retaining the aqueous solution inside the recesses. Due to the effect of fluid shear force, an interface between the aqueous solution and air is formed in the hollow portion of the opening member. Then, 5 µL of a non-polar medium solution (n-hexadecane) in which amphipathic molecules (1,2-bis(diphenylphosphine)ethane (DPPE) phospholipid molecules) (50 mg/mL) are dissolved, and 1 mL of an aqueous solution of potassium chloride (500 mM) and Hepes (5 mM), as the second polar medium solution, are pushed into sequentially.

Due to the effect of the surface tension of the liquid, the non-polar medium solution and the amphipathic molecules dissolved in it are naturally laid flat in the hollow portion of the opening member and completely cover the first polar medium solution located in the hollow portion, thus forming a layer of water/oil/water interface in the hollow portion. Phospholipid molecules spontaneously arrange themselves at the interface between the aqueous solution and oil (n-hexadecane) through self-assembly. Finally, under the intermolecular force, the phospholipid molecules in some areas self-assemble to form a bilayer phospholipid membrane. As time goes by, the membrane area becomes larger and larger and trended to be stable.

The object represented in Fig. 22 is an array of carrier membranes formed on an array of a plurality of recesses with an opening diameter of about 80 µm. The fluorescence confocal microscope (FCFM) model used in the experiment is Nikon C2+. The first polar medium solution in the accommodating chamber in each recess contains 0.02% by mass of Rhodamine B fluorescent dye. The excitation peak wavelength and emission peak wavelength of Rhodamine B are at 546 nm and 568 nm, respectively. The second polar medium solution above the hollow portion of the opening member does not contain Rhodamine B. 2 µM BODIPY FL fluorescent dye is mixed into the non-polar medium solution. The excitation peak and emission peak of BODIPY FL are at 503 nm and 512 nm, respectively. Since BODIPY FL is easily soluble in non-polar medium solutions, the areas containing non-polar medium solutions in the array device appear green.

In the microscope phenogram shown in Fig. 22, Rhodamine B is dissolved in the aqueous solution below the membrane and appears red in the phenogram (indicated by arrow R). The aqueous solution above the membrane does not contain Rhodamine B and therefore has no fluorescence, showing a black background. The non-polar medium solution containing phospholipids appears light green in the phenogram (indicated by arrow G) . As can be seen from the fluorescence confocal cross-section shown in Fig. 22, at the membrane interface, the first polar medium solution inside the accommodating chambers of the recesses presents a slightly convex shape, indicating that the actual shapes of the carrier membranes are slightly convex curved surfaces. As can be seen from the top view shown in Fig. 22, the boundary of the carrier membrane is a relatively regular circle.

Fig. 23 is a confocal fluorescence microscopy phenogram of a membrane array in a second embodiment of the membrane forming method according to the present disclosure. Referring to Fig. 16, in the second embodiment of the membrane forming method, an aqueous solution of potassium chloride (500 mM) and Hepes (5mM), as the first polar medium solution, is introduced into the flow channel of the membrane forming device including a plurality of recesses ((b) of Fig. 11) and the aqueous solution fills the accommodating chamber. The aqueous solution also contains 0.02% by mass of Rhodamine B. Then, 10 mL of air is pushed in from the flow channel of the membrane forming device to completely discharge the aqueous solution directly above the main body and the opening member, while retaining the aqueous solution inside the recesses. Due to the effect of fluid shear force, an interface between the aqueous solution and air is formed in the hollow portion of the opening member.

Then, the sealing cover plate is removed, and 30 µL of the non-polar medium solution (n-hexadecane) in which 50 mg/mL amphipathic molecules (1,2-bis(diphenylphosphine)ethane (DPPE) phospholipid molecules) are dissolved is then dropwise added directly above the main body and wait for 10 min. At this time, the phospholipid molecules self-assemble at the water-oil interface through molecular forces to form a stable phospholipid monolayer. Then, the sealing cover plate is assembled, and 1 mL of the second polar solution without Rhodamine B is pushed in from the flow channel of the membrane forming device. Then, wait for 24 h without disturbance, during which time the membrane will self-assemble to form a stable phospholipid bilayer.

After the carrier membrane is stable, 5 mL of 500 mM potassium chloride and 5 mM Hepes solution without Rhodamine is pushed in from the liquid inlet for flushing. Referring to Fig. 23, the circular fluorescent spots appearing in the microscope phenogram represent the formation of a stable carrier membrane. If the membrane formation fails, after the membrane ruptures, it will fail to block the diffusion of the fluorescent solvent at the bottom of the recess. The fluorescent dye in the accommodating chambers of the recesses will quickly diffuse into the non-fluorescent second polar solution outside the accommodating chambers, eventually appearing gray-black in fluorescence microscope phenogram. In Fig. 23, there is strong fluorescence at the position corresponding to each recess, which shows that the membrane formation yield reaches 100%.

Figs. 24 and 25 are respectively graphs showing transmembrane current curves monitored when triangular wave voltage is applied to different channels after membrane formation in embodiments of the membrane forming device according to the present disclosure. Based on the membrane forming device with a hollow portion having a diameter of about 47 µm as shown in Fig. 19, after the membrane is formed by the membrane forming method disclosed herein, the carrier membrane is stabilized for 24 h, and then a triangular wave voltage with an amplitude of 50 mV is applied to the electrodes on both sides of the carrier membrane, and the current is monitored in real time.

Referring to Fig. 24, when an independent channel is driven by the triangular wave voltage, the transmembrane current appears as a quasi-square wave, with a peak value of about 80-90 pA. Then, the membrane capacitance is calculated to be 40 pF, which is within the reasonable capacitance range (10-150 pF) for normal membrane formation. Referring to Fig. 25, when another independent channel is driven by the triangular wave voltage, the transmembrane current appears as a quasi-square wave, with a peak value of about 60-70 pA. Then, the membrane capacitance is calculated to be 30.7 pF, which is within the reasonable capacitance range (10-150 pF) for normal membrane formation.

Fig. 26 is a graph showing a transmembrane current curve monitored when triangular wave voltage is applied to after membrane formation in an embodiment of the membrane forming device according to the present disclosure. Based on the membrane forming device with a hollow portion having a diameter of about 90 µm, after the membrane is formed by the membrane forming method disclosed herein, the carrier membrane is stabilized for 24 h, and then a triangular wave voltage with an amplitude of 50 mV is applied to the electrodes on both sides of the carrier membrane, and the current is monitored in real time. Referring to Fig. 26, driven by the triangular wave voltage, the transmembrane current appears as a quasi-square wave, with a peak value of about 200-220 pA. Then, the membrane capacitance is calculated to be 100 pF, which is within the reasonable capacitance range (10-150 pF) for normal membrane formation.

In another embodiment, the membrane forming device with a hollow portion having a diameter of about 80 µm as shown in Fig. 18 is used, and capacitance statistics are performed after the membrane is formed by the membrane forming method of the present disclosure. Just after the membrane is formed, because there is still a relatively large amount of non-polar medium solution in the membrane, the membrane thickness is large or the membrane area is small, so the membrane capacitance of most channels is measured to be between 0 and 20 pF. After 24 h, the capacitance is monitored again. The membrane capacitance of most channels is increased to about 30-40 pF and tended to be stable. It can be seen that the values of membrane capacitance are all within the reasonable capacitance range for normal membrane formation (10-150 pF). After monitoring, it is found that only one channel out of 128 channels has membrane capacitance greater than 300 pF for the reason determined as short circuit in that channel due to membrane rupture, while the remaining 127 channels all has intact carrier membranes, so the membrane formation yield is calculated as 127/128=99.2%. Therefore, not only the membrane formation yield is very high, but the stability of the carrier membrane is also very good.

Fig. 27 is a graph showing the capacitance of multiple channels changing with time during the membrane forming process in an embodiment of the membrane forming device according to the present disclosure. A membrane forming device with a hollow portion having a diameter of about 110 µm is used. The membrane forming device uses silver/silver chloride electrodes. After the membrane is formed using the membrane forming method described herein, the curves of the membrane capacitance of multiple independent channels in the membrane forming device are obtained through long-term measurements. The data recording mode for membrane capacitance is to record the capacitance of all channels every ten minutes. Referring to Fig. 27, the x-axis represents time in the smallest unit of an hour (h), and the y-axis represents capacitance in the unit of picofarad (pF). Fig. 27 records the capacitance data of multiple independent channels for a total of 15 hours after membrane formation. As can be seen from the data, during the initial stage of membrane formation, the capacitance of most channels is between 0 and 20 pF. Afterwards, the carrier membrane spontaneously thins through the van der Waals force between molecules, and as a result the area of the carrier membrane continues to increase and the capacitance gradually increases.

The effective area of the membrane can be estimated by referring to the membrane capacitance calculation formula of the parallel plate capacitor: C=(ε₀*εᵣ*S)/d, where C represents membrane capacitance, ε₀ represents the vacuum dielectric constant, εᵣ represents the dielectric constant of membrane molecules, S represents the effective area of the membrane, and d represents the thickness of the membrane. Generally, in a case where amphipathic molecules self-assemble to form a stable monolayer or bilayer membrane, the thickness d of the membrane and the dielectric constant of the membrane molecules remain constant, while the vacuum dielectric constant ε₀ is a constant, so the membrane capacitance C is directly in linear dependence to the effective area S of the membrane. The larger the effective area of the membrane, the larger the capacitance; conversely, the smaller the effective area of the membrane, the smaller the capacitance. The capacitance of most channels hops rapidly and stabilizes to around 110 pF from the 1st hour to the 10th hour after membrane formation. This capacitance value is within the reasonable capacitance value range (10-150 pF) for normal membrane formation. Only a few channels still do not achieve stable capacitance.

Fig. 28 is a schematic diagram of detection current signals when single molecules are detected by an electrical method using a carrier membrane formed by an embodiment of the membrane forming device according to the present disclosure. The carrier membrane obtained by the membrane forming device and membrane forming method described above can be used for the detection of single molecules, for example, by electrical methods. After a stable carrier membrane is formed, 200 µL of a solution containing nanopore protein (MspA) is introduced into the flow channel. Nanopore proteins are randomly distributed near the carrier membrane due to the flow of the solution and the thermal motion of molecules. Then, under the stimulation of voltage, a single nanopore protein successfully crosses the barrier of molecules in the carrier membrane and is successfully embedded into the carrier membrane. After the nanopore protein is successfully embedded, a solution containing the nucleic acid molecules to be tested is added to a flow channel inlet of the membrane forming device, and then a voltage of 180mV is applied to the electrodes of the membrane firming device. A single nucleic acid molecule passes through the nanopore protein under the action of electric field force and forms a continuous ionic current blocking signal. Referring to Fig. 28, when nucleic acid molecules pass through the nanopores, they generate a fluctuating signal with a current of about 50-150 pA.

(a) and (b) of Fig. 29 are dimensioned schematic diagrams of a membrane forming device in the related art and an embodiment of the membrane forming device according to the present disclosure. In conventional electrochemical detection, consumable electrodes, such as silver/silver chloride electrodes, are usually used. In this case, it is usually necessary to focus on the service life of the electrode itself, instead of the volume of the electrolyte inside the accommodating chamber of the membrane forming device and the consumption of the solution.

However, in another electrochemical detection system, the detection electrodes may be non-consumable electrodes (such as gold or platinum and other inert metal electrodes), and correspondingly, the solution in the accommodating chamber of the membrane forming device needs to be replaced with a consumable electronic pair solution (such as potassium ferricyanide/potassium ferrocyanide solution). In this case, limited by the need to increase the array density and the number of detection channels, the size of the recesses is usually designed to be very small, and volume of the solution inside the accommodating chamber are of very small amount (usually in the nanoliter scale). Therefore, it is necessary to focus on the impact of the internal volume of the recesses on the detection life. Referring to (a) and (b) of Fig. 29, calculations and comparisons are made between the total volume of the solution inside the accommodating chamber in the related art shown in (a) of Fig. 29 and the total volume of the solution inside the accommodating chamber in the embodiment of the membrane forming device according to the present disclosure shown in (b) of Fig. 29. It can be understood that in the case of non-consumable electrodes and consumable electron pair solutions, the accommodating chamber in the embodiment of the membrane forming device according to the present disclosure has great advantages.

Referring to (a) and (b) of Fig. 29, it is assumed that the accommodating chamber of the related art and the accommodating chamber in the embodiment of the membrane forming device according to the present disclosure are both in the shape of a cylinder. The corresponding cylinder of the accommodating chamber in the related art has a diameter of d0 and a height of h₀. In the embodiment of the membrane forming device according to the present disclosure, the corresponding cylinder of the accommodating chamber has a diameter of d₁ and a height of h₁, the opening member has a height of h₂, and the hollow portion of the opening member has a diameter of d₂.

According to the volume formula, the internal solution volume of the accommodating chamber in the related art can be calculated as: V₀=(π*d₀²*h₀)/4, and the internal solution volume of the accommodating chamber in the embodiment of the membrane forming device according to the present disclosure is calculated as: V₁≈(π*d₁²*h₁)/4. Under the condition of ensuring effective reduction of membrane area (d₂<d₀), given that d₁=2*d₀, h₁=h₀, then V₁≈(π*d₁²*h₁)/4=4* ((π*d₀²*h₀)/4)=4*V₀, that is, the internal solution volume of the accommodating chamber in the embodiment of the membrane forming device according to the present disclosure is approximately 4 times the internal solution volume of the accommodating chamber in the related art.

Taking typical values d₀=100 pm, d₁=200 pm, h₀=100 pm, h₁=100 µm, then we have V₀=7.85×10⁵ µm³, V₁=3.14×10⁶ µm³. V₀ is approximately equal to 0.8 nL, and V₁ is approximately equal to 3.1 nL. It can be seen that the embodiment of the membrane forming device according to the present disclosure has great advantages compared with the related art. The storage capacity of the solution is four times or more that of the related art, thereby ensuring a longer detection life.

The data presented in the various experiments described above are technically interoperable and interrelated. For the carrier membrane formed, the measured capacitance of the carrier membrane is within a reasonable range (10-150 pF), which means normal membrane formation. Therefore, the transmembrane current curves with hollow portions having diameters of about 47 µm and 90 µm shown in Fig. 24 to Fig. 26 respectively are used to present the single-channel capacitance measurement results. The capacitance change diagram with the hollow portion having a diameter of about 110 µm, as shown in Fig. 27, presents the dynamic change process of the capacitance from the beginning of membrane formation to the final formation of a stable membrane. The membrane formation yield obtained from multi-channel statistics of a membrane forming device with a hollow part having a diameter of about 80 µm is also provided above.

So far, various embodiments of the present disclosure have been described in detail. In order to avoid obscuring concepts of the present disclosure, some details that are well known in the art have not been described. Those skilled in the art can fully understand how to implement the technical solutions disclosed here according to the above descriptions.

Although some specific embodiments of the present disclosure have been described in detail through examples, it should be understood by those skilled in the art that the above examples are only for illustration and are not intended to limit the scope of the present disclosure. Those skilled in the art should understand that the above embodiments can be modified or some technical features can be replaced by equivalents without departing from the scope and spirit of the present disclosure. The scope of the present disclosure is defined by appended claims.

## Claims

1. A membrane forming device, comprising:
a base (10);
a main body (20) arranged to the base (10) and the main body (20) having a recess; and
an opening member (30) arranged inside a recess port on an end of the recess away from the base (10), and a hollow portion (32) of the opening member (30) being configured to form a membrane (80) .

2. The membrane forming device according to claim 1, wherein the recess is configured to define an accommodating chamber (21) for accommodating a polar medium solution, the membrane (80) is an amphipathic molecular membrane, and a cross-sectional area of the hollow portion (32) of the opening member (30) is smaller than that of the accommodating chamber (21).

3. The membrane forming device according to any one of the preceding claims, wherein the main body (20) comprises:
a first body layer (22) arranged to a surface of the base (10); and
a second body layer (23) located on a side of the first body layer (22) away from the base (10),
wherein the recess penetrates the second body layer (23) and the first body layer (22) in a direction perpendicular to the base (10), and the opening member (30) is connected to a portion of the recess penetrating the second body layer (23).

4. The membrane forming device according to any one of the preceding claims, wherein the opening member (30) is integrally formed with the second body layer (23).

5. The membrane forming device according to any one of the preceding claims, wherein the opening member (30) is integrally formed with the main body (20).

6. The membrane forming device according to any one of the preceding claims, wherein the opening member (30) comprises:
a flange (31) protruding radially inwardly relative to the recess port,
wherein an inner edge of the flange (31) forms the hollow portion (32).

7. The membrane forming device according to any one of the preceding claims, wherein the inner edge of the flange (31) is located on a same plane.

8. The membrane forming device according to any one of the preceding claims, wherein a cantilevered end of the flange (31) is chamfered or rounded.

9. The membrane forming device according to any one of the preceding claims, wherein a section of the flange (31) is T-shaped or in a shape of a convex polygon.

10. The membrane forming device according to any one of the preceding claims, wherein a thickness of the cantilevered end of the flange (31) in a direction perpendicular to the base (10) is equal to or less than a thickness of a root portion of the flange (31) in the direction perpendicular to the base (10).

11. The membrane forming device according to any one of the preceding claims, wherein a thickness of the flange (31) in the direction perpendicular to the base (10) gradually decreases from the root portion to the cantilevered end.

12. The membrane forming device according to any one of the preceding claims, wherein the flange (31) has at least one portion that protrudes or recesses from a surface of the flange (31) in a direction perpendicular to the base (10).

13. The membrane forming device according to any one of the preceding claims, wherein the at least one portion is at least located on a side of the flange (31) away from the base (10).

14. The membrane forming device according to any one of the preceding claims, wherein the inner edge of the flange (31) has a plurality of protrusions protruding inwardly.

15. The membrane forming device according to any one of the preceding claims, wherein the cross section of the hollow portion (32) is in a shape of a circle or a convex polygon, and the cross section of the accommodating chamber (21) is in a shape of a circle or a convex polygon.

16. The membrane forming device according to any one of the preceding claims, wherein a longitudinal cross section of the accommodating chamber (21) is in a shape of a rectangle, a trapezoid, a curved-edge trapezoid, or a drum.

17. The membrane forming device according to any one of the preceding claims, further comprising:
a first electrode (51) arranged outside the accommodating chamber (21); and
a second electrode (52) arranged inside the accommodating chamber (21).

18. The membrane forming device according to any one of the preceding claims, wherein the base (10) comprises:
a substrate (11); and
at least one passivation layer (12, 13), arranged between the substrate (11) and the main body (20),
wherein the second electrode (52) is exposed from a bottom of the recess; and
the second electrode (52) is located between the substrate (11) and the at least one passivation layer (12, 13), or the second electrode (52) is located on a side of the at least one passivation layer (12, 13) away from the substrate (11), or the second electrode (52) is located between adjacent passivation layers (12, 13) in a case where the at least one passivation layer (12, 13) is configured as a plurality of passivation layers (12, 13).

19. The membrane forming device according to any one of the preceding claims, wherein the main body (20) comprises a plurality of recesses, and the plurality of recesses of the main body (20) are arranged in a rectangular array, a parallelogram array, a honeycomb array, or irregularly.

20. The membrane forming device according to any one of the preceding claims, wherein the opening member (30) has a plurality of hollow portions (32).

21. The membrane forming device according to any one of the preceding claims, further comprising:
a sealing cover plate (60) configured to be assembled with the main body (20).

22. The membrane forming device according to any one of the preceding claims, further comprising:
a sealing cover plate (60) configured to be assembled with the main body (20);
an amphipathic molecular membrane (80) formed in the hollow portion (32) of the opening member (30);
a first polar medium solution (M1) accommodated in the accommodating chamber (21), and
a second polar medium solution (M4) accommodated outside the accommodating chamber (21) and located above the main body (20), the opening member (30) and the amphipathic molecular membrane (80) .

23. The membrane forming device according to any one of the preceding claims, wherein the amphipathic molecular membrane (80) is a monolayer lipid membrane, a bilayer lipid membrane or a high molecular polymer membrane.

24. The membrane forming device according to any one of the preceding claims, further comprising:
a membrane channel (90) embedded into the amphipathic molecular membrane (80).

25. A membrane forming method, comprising:
providing the membrane forming device according to any one of the preceding claims, and assembling a sealing cover plate (60) to an upper side of the main body (20) to form a flow channel between the sealing cover plate (60) and the main body (20);
introducing a first polar medium solution (M1) into the flow channel so that the first polar medium solution (M1) flows into a recess of the main body (20) from a hollow portion (32) of an opening member (30) and fills the recess of the main body (20); and
removing the first polar medium solution (M1) between the opening member (30) and the sealing cover plate (60), and causing a non-polar medium solution (M3) and a second polar medium solution (M4) to cover the opening member (30),
wherein at least one of the non-polar medium solution (M3) and the second polar medium solution (M4) contains amphipathic molecules to form an amphipathic molecular membrane (80) in the hollow portion (32).

26. The membrane forming method according to any one of the preceding claims, wherein the step of removing the first polar medium solution (M1) between the opening member (30) and the sealing cover plate (60) comprises:
introducing a gas medium (M2) into the flow channel so as to remove the first polar medium solution (M1) between the main body (20) and the sealing cover plate (60) and between the opening member (30) and the sealing cover plate (60) through the gas medium (M2) .

27. The membrane forming method according to any one of the preceding claims, wherein the step of causing the non-polar medium solution (M3) and the second polar medium solution (M4) to cover the opening member (30) comprises:
introducing the non-polar medium solution (M3) into the flow channel so that the non-polar medium solution (M3) covers the opening member (30); and
introducing the second polar medium solution (M4) into the flow channel so that the second polar medium solution (M4) covers the opening member (30).

28. The membrane forming method according to any one of the preceding claims, wherein the step of causing the non-polar medium solution (M3) and the second polar medium solution (M4) to cover the opening member (30) comprises:
removing the sealing cover plate (60);
coating the non-polar medium solution (M3) to the main body (20) and the opening member (30) and causing the non-polar medium solution (M3) to cover the opening member (30);
assembling the sealing cover plate (60) to the upper side of the main body (20) to form a flow channel between the sealing cover plate (60) and the main body (20); and
introducing the second polar medium solution (M4) into the flow channel so that the second polar medium solution (M4) covers the opening member (30).

29. The membrane forming method according to any one of the preceding claims, wherein the step of removing the first polar medium solution (M1) between the opening member (30) and the sealing cover plate (60), and causing the non-polar medium solution (M3) and the second polar medium solution (M4) to cover the opening member (30) comprises:
introducing the non-polar medium solution (M3) into the flow channel so as to remove a first polar medium solution (M1) between the main body (20) and the sealing cover plate (60) and a first polar medium solution (M1) between the opening member (30) and the sealing cover plate (60) by virtue of the non-polar medium solution (M3), and to achieve coverage of the opening member (30) by the non-polar medium solution (M3); and
introducing the second polar medium solution (M4) into the flow channel so that the second polar medium solution (M4) covers the opening member (30).

30. The membrane forming method according to any one of the preceding claims, further comprising:
embedding a membrane channel (90) into the amphipathic molecular membrane (80).
